(19) Europäisches Patentamt
European Patent Office
Office européen des brevets

(11) Publication number: 0 132 278 B1

(12) EUROPEAN PATENT SPECIFICATION

(45) Date of publication of patent specification: 10.08.88

(51) Int. Cl.[4]: C 07 D 309/32, C 07 D 405/06, A 01 N 43/16, A 01 N 43/40, A 01 N 43/60, A 01 N 43/84

(21) Application number: 83900354.8

(22) Date of filing: 17.01.83

(86) International application number: PCT/JP83/00013

(87) International publication number: WO 84/02910 02.08.84 Gazette 84/18

# (54) PYRAN DERIVATIVES.

(43) Date of publication of application: 30.01.85 Bulletin 85/05

(45) Publication of the grant of the patent: 10.08.88 Bulletin 88/32

(84) Designated Contracting States: FR GB

(56) References cited:
JP-A-52 153 967
JP-A-55 055 177
JP-A-55 055 178
JP-A-55 055 179
JP-A-58 010 573
US-A-3 751 434
US-A-4 289 704

No relevant documents have been disclosed

The file contains technical information submitted after the application was filed and not included in this specification

(73) Proprietor: OTSUKA KAGAKU YAKUHIN KABUSHIKI KAISHA
10, Bungomachi Higashi-ku
Osaka-shi Osaka 540 (JP)

(72) Inventor: TAKAO, Hisashi Otsuka Kagaku Yakuhin K. K.
Naruto Factory 649-2, Aza-Hanamen, Satoura
Satoura-cho,Naruto-shi Tokushima-ken 772 (JP)
Inventor: OSAKI, Norio Otsuka Kagaku Yakuhin K. K.
Naruto Factory 649-2, Aza-Hanamen, Satoura
Satoura-cho,Naruto-shi Tokushima-ken 772 (JP)
Inventor: YASUTOMI, Norio Otsuka Kagaku Yakuhin K. K.
NarutoFactory 649-2, Aza-Hanamen, Satoura
Satoura-cho,Naruto-shi Tokushima-ken 772 (JP)

(74) Representative: Barz, Peter, Dr.
Patentanwälte Dr. V. Schmied-Kowarzik Dipl.-Ing. G. Dannenberg Dr. P. Weinhold Dr. D. Gudel Dipl.-Ing. S. Schubert Dr. P. Barz
Siegfriedstrasse 8
D-8000 München 40 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 132 278 B1

**Description**

The invention relates to novel pyran derivatives, a process for their preparation and a miticide containing these pyran derivatives.

Pyran derivatives having miticidal activity are disclosed in JP—A—55—55177, 55—55178 and 55—55179.

The pyran derivatives of the present invention are represented by the following general formula

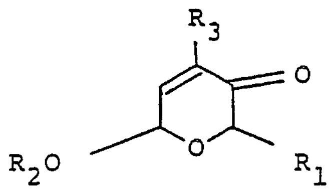

(I)

wherein $R_1$ is hydrogen, lower alkyl of 1 to 6 carbon atoms, cycloalkyl of 3 to 7 carbon atoms or phenyl, $R_2$ is alkyl of 8 to 20 carbon atoms, $R_3$ is hydrogen, halogen,

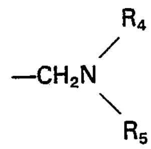

or

$—CN_2NA$,

$R_4$ and $R_5$ being each lower alkyl of 1 to 6 carbon atoms or cycloalkyl of 3 to 7 carbon atoms, A being $—CH_2—$, an oxygen or nitrogen atom,

NA

forms a five or six-membered ring (the ring may have no substituent or have at least one substituent selected from lower alkyl of 1 to 6 carbon atoms, lower alkoxy of 1 to 6 carbon atoms and halogen).

In a preferred group of compounds $R_3$ is

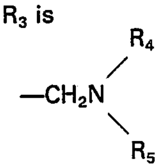

,

$R_4$ and $R_5$ being each lower alkyl of 1 to 6 carbon atoms.

In another preferred group of compounds $R_3$ is

$—CH_2NB$,

B being $—CH_2—$ or an oxygen atom.

In the above general formula (I), examples of lower alkyl are methyl, ethyl, n-propyl, isopropyl, n-butyl, isobutyl, pentyl and hexyl. Examples of cycloalkyl are cyclopropyl, cyclobutyl, cyclopentyl, cyclohexyl and cycloheptyl. Examples of alkyl having 8 to 20 carbon atoms are octyl, decyl, undecyl, tetradecyl, octadecyl and eicosyl. Halogen atoms include e.g. chlorine and bromine.

It has been reported that compounds analogous to the pyran derivative of the above formula [1] of the invention were useful as intermediates of maltol derivatives [JP—A—12 166/1977 and 18 578/1978].

The pyran derivatives of the present invention are different from the above known compounds in a substituent on the pyran ring, and have acaricide action which are not disclosed in the above prior references. The present compounds are low in toxicity and are especially useful as agricultural miticides.

Representative pyran derivatives of the formula [1] having acaricide action are as follows:

6-Lauryloxy-2H-pyran-3(6H)-one
6-Lauryloxy-2-methyl-2H-pyran-3(6H)-one
6-Lauryloxy-2-ethyl-2H-pyran-3(6H)-one
6-Decyloxy-2-n-propyl-2H-pyran-3(6H)-one
6-Lauryloxy-2-n-propyl-2H-pyran-3(6H)-one
6-Cetyloxy-2-n-propyl-2H-pyran-3(6H)-one
6-Octyloxy-2-isopropyl-2H-pyran-3(6H)-one
6-Decyloxy-2-isopropyl-2H-pyran-3(6H)-one
6-Lauryloxy-2-isopropyl-2H-pyran-3(6H)-one
6-Cetyloxy-2-isopropyl-2H-pyran-3(6H)-one

6-Lauryloxy-2-cyclopropyl-2H-pyran-3(6H)-one
6-Cetyloxy-2-cyclopropyl-2H-pyran-3(6H)-one
6-Lauryloxy-2-n-butyl-2H-pyran-3(6H)-one
6-Octyloxy-2-isobutyl-2H-pyran-3(6H)-one
6-Lauryloxy-2-isobutyl-2H-pyran-3(6H)-one
6-Lauryloxy-2-n-amyl-2H-pyran-3(6H)-one
6-Octyloxy-2-n-hexyl-2H-pyran-3(6H)-one
6-Lauryloxy-2-phenyl-2H-pyran-3(6H)-one
6-Octyloxy-2-cyclohexyl-2H-pyran-3(6H)-one
6-Lauryloxy-2-cyclohexyl-2H-pyran-3(6H)-one
6-Decyloxy-4-chloro-2-n-propyl-2H-pyran-3(6H)-one
6-Lauryloxy-4-bromo-2-n-propyl-2H-pyran-3(6H)-one
6-Cetyloxy-4-chloro-2-n-propyl-2H-pyran-3(6H)-one
6-Cetyloxy-4-bromo-2-n-propyl-2H-pyran-3(6H)-one
6-Octyloxy-4-chloro-2-isopropyl-2H-pyran-3(6H)-one
6-Lauryloxy-4-chloro-2-isopropyl-2H-pyran-3(6H)-one
6-Cetyloxy-4-chloro-2-isopropyl-2H-pyran-3(6H)-one
6-Cetyloxy-4-bromo-2-isopropyl-2H-pyran-3(6H)-one
6-Cetyloxy-4-chloro-2-cyclopropyl-2H-pyran-3(6H)-one
6-Cetyloxy-4-bromo-2-cyclohexyl-2H-pyran-3(6H)-one
6-Lauryloxy-4-chloro-2-isobutyl-2H-pyran-3(6H)-one
6-Lauryloxy-4-di-n-butylaminomethyl-2-n-propyl-2H-pyran-3(6H)-one
6-Cetyloxy-4-diethylaminomethyl-2-n-propyl-2H-pyran-3(6H)-one
6-Cetyloxy-4-piperidinomethyl-2-n-propyl-2H-pyran-3(6H)-one
6-Cetyloxy-4-(4-methylpiperidinomethyl)-2-n-propyl-2H-pyran-3(6H)-one
6-Octyloxy-4-di-n-butylaminomethyl-2-isopropyl-2H-pyran-3(6H)-one
6-Octyloxy-4-piperidinomethyl-2-isopropyl-2H-pyran-3(6H)-one
6-Lauryloxy-4-piperidinomethyl-2-isopropyl-2H-pyran-3(6H)-one
6-Cetyloxy-4-diethylaminomethyl-2-isopropyl-2H-pyran-3(6H)-one
6-Cetyloxy-4-di-n-butylaminomethyl-2-isopropyl-2H-pyran-3(6H)-one
6-Cetyloxy-4-piperidinomethyl-2-isopropyl-2H-pyran-3(6H)-one
6-Cetyloxy-4-(4-methylpiperidinomethyl)-2-isopropyl-2H-pyran-3(6H)-one
6-Cetyloxy-4-morpholinomethyl-2-isopropyl-2H-pyran-3(6H)-one
6-Cetyloxy-4-N-methylpiperazinomethyl-2-isopropyl-2H-pyran-3(6H)-one
6-Cetyloxy-4-pyrrolidinomethyl-2-isopropyl-2H-pyran-3(6H)-one
6-Lauryloxy-4-piperidinomethyl-2-cyclopropyl-2H-pyran-3(6H)-one
6-Cetyloxy-4-piperidinomethyl-2-cyclopropyl-2H-pyran-3(6H)-one
6-Cetyloxy-4-(4-methylpiperidinomethyl)-2-cyclopropyl-2H-pyran-3(6H)-one
6-Lauryloxy-4-piperidinomethyl-2-isobutyl-2H-pyran-3(6H)-one
6-Lauryloxy-4-piperidinomethyl-2-cyclohexyl-2H-pyran-3(6H)-one

The present pyran derivatives of the formula [1] are prepared, for example, by the following reaction equations 1 to 3.

[Reaction equation 1]

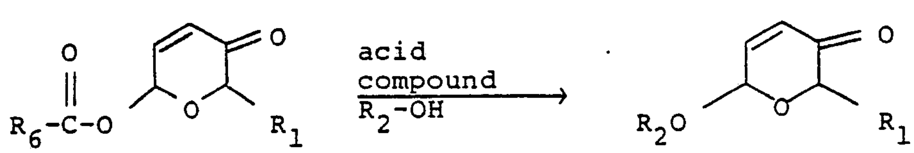


[ 2 ] [ 3 ]

wherein $R_1$ and $R_2$ are as defined above, $R_6$ is a lower alkyl group of 1 to 6 carbon atoms.

In the above, the starting pyran derivative [2] is a known compound and is prepared according to a disclosure in JP—A—128876/1978. The reaction of the derivative [2] with the alcohol is conducted in the presence of an acid compound in a solvent. Examples of useful alcohols are octyl alcohol, 2-ethylhexyl alcohol, nonyl alcohol, decyl alcohol, lauryl alcohol, cetyl alcohol, stearyl alcohol and eicosyl alcohol. Examples of useful solvents include diethyl ether, tetrahydrofuran, dioxane and like ethers, benzene, toluene, xylene and like aromatic hydrocarbons, dichloromethane, chloroform, carbon tetrachloride and like hydrocarbon halides. Examples of useful acid compounds are stannic chloride, zinc chloride, boron fluoride and like Lewis acids, trifluoroacetic acid, p-toluenesulfonic acid and like organic acids, sulfuric acid, hydrochloric acid, phosphoric acid and like inorganic acids. The alcohols are used in an amount of usually

about 0.5 to 3 moles, preferably about 1 to 1.2 moles per mole of the starting pyran derivative [2]. The acid compounds are used in an amount of usually about 0.001 to 0.1 mole, preferably about 0.05 to 0.1 mole per mole of the starting derivative [2]. The reaction is completed at a temperature of usually −30 to 40°C, preferably 0°C to room temperature in about 3 to 10 hours. In the above, the present compound of the formula [1] wherein $R_3$ is a hydrogen atom (compound [3]) is obtained.

[Reaction equation 2]

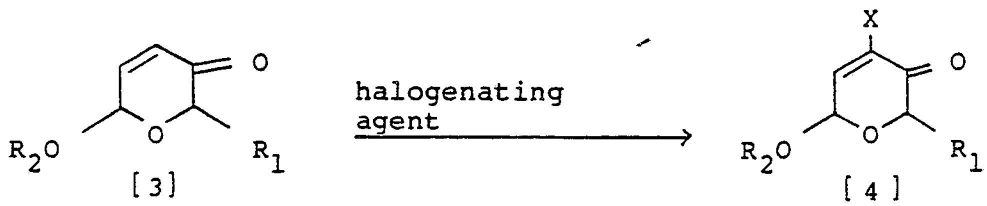

[3] [4]

wherein $R_1$ and $R_2$ are as defined above, X is a halogen atom.

In the above, the starting pyran derivative [3] is obtained according to the above reaction equation 1. The reaction of the compound [3] with a halogenating agent is conducted preferably in a solvent such as a beforementioned hydrocarbon halide or ether, at a temperature of −30 to 50°C, preferably 0°C to room temperature for about 30 minutes to 2 hours. The reaction is completed by adding a basic compound and heating the system at a same temperature for about 1 to 3 hours. Halogenating agents include chlorine, bromine, iodine and like halogen atoms, hypochlorous acid, hypobromous acid, hypoiodous acid and like hypohalogenous acids, and are used in an amount of at least one mole, preferably 1 to 1.2 moles per mole of the starting compound. Basic compounds include triethylamine, tripropylamine, pyridine and like tertiary amines, sodium hydroxide, potassium hydroxide, sodium carbonate, potassium carbonate and like inorganic bases, and are used in an amount of usually at least one mole, preferably 1 to 2 moles per mole of the starting compound. Thus, the present compound of the formula [1] wherein $R_3$ is a halogen atom (compound [4]) is obtained.

[Reaction equation 3]

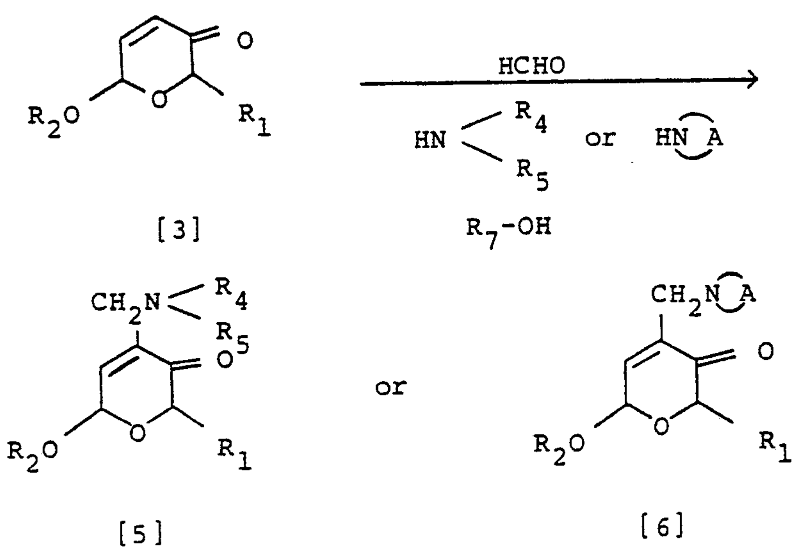

[3]

[5] or [6]

wherein $R_1$, $R_2$, $R_4$, $R_5$ and A are as defined above, $R_7$ is a lower alkyl of 1 to 6 carbon atoms.

The aminomethylation of the compound [3], formaldehyde and amine is conducted preferably in a lower alcohol. Examples of useful lower alcohols are methanol, ethanol, propanol and butanol. Each of formaldehyde and amine is used in an amount of about 0.5 to 3 moles, preferably about 1 to 1.5 moles per mole of the starting compound [3]. The reaction is conducted at a temperature of usually room temperature to 100°C, preferably about 40 to 60°C for about 3 to 10 hours. The desired compound [5] or [6] is isolated in the form of an acid salt thereof, when desired. Thus, the present compound of the formula [1] in which $R_3$ is aminomethyl (compound [5] or [6]) is obtained.

The present compound of the formula [1] can be prepared according to the above reaction equations 1 to 3, and can easily be separated and purified by a usual method such as solvent extraction, solvent dilution, distillation, recrystallization or column chromatography. The present pyran derivative is a novel

compound which does not resemble to commercially available miticides, and is highly estimated for totally preventing mites due to its long lasting miticide activity.

The present miticide composition contains the pyran derivative of the formula [1] as an effective component. The composition is formulated into desired preparations as similar to usual insecticides. For example, the composition can be in any of various forms such as particle, powder, emulsion, dispersion, aqueous solution, tablet, oily solution, spray or aerosol, in combination with suitable solid carrier, liquid carrier, suspending agent or spreader. Examples of useful carriers are clay, kaolin, bentonite, talc, terra abla, diatomaceous earth, calcium carbonate, nitrocellulose, starch, gum arabic, carbon dioxide, fluorinated hydrocarbon, water, benzene, kerosene, alcohol, acetone, xylene, methylnaphthalene, cyclohexanone, animal or vegetable fatty acid esters. Examples of suspending agents and spreaders are usual surfactants such as soaps, higher alcohol sulfate esters, alkylsulfonic acid salts, quaternary ammonium salts and polyalkylene oxides.

The amount of the present compound of the formula [1] to be contained in the miticide of this invention can be suitably determined according to forms used. For example, in the form of dispersion or aqueous solution, the amount is usually about 0.1 to 90% by weight of the whole composition. In the form of powder or oily solution, the amount is usually about 0.1 to 10% by weight.

The present miticide composition can be applied by spreading, spraying, coating and the like to places requiring sterilization effects as similar to known insecticides. The amount to be applied can be suitably determined but is usually about 0.1 to 10 kg, preferably about 0.1 to 5 kg of the effective component per one hectare for agricultural and horticultural uses. The amount can be suitably increased or decreased depending on the kinds of plants or the symptoms to be treated. The present miticide composition can be used in combination with e.g. other miticides, germicides, herbicides, fetilizers and soil improving agents.

The invention will be described below in greater detail with reference to the following preparation examples and test examples.

Preparation Example 1

In 50 ml of ethyl ether were dissolved 3.96 g of 6-acetoxy-2-isopropyl-2H-pyran-3(6H)-one and 4.84 g of cetyl alcohol with stirring. Into the solution was gradually added 0.1 ml of anhydrous stannic chloride at a temperature of 5 to 10°C. The reaction mixture was stirred at room temperature for 7 hours, washed twice with aqueous sodium hydrogencarbonate solution and once with saturated aqueous sodium chloride solution and then dried over anhydrous magnesium sulfate. By the distillation of solvent was obtained 7.0 g of 6-cetyloxy-2-isopropyl-2H-pyran-3(6H)-one [compound 1] in a yellow viscous oily form.

IR (neat, $cm^{-1}$); 1698 (C = O), 1628 (C = C)
NMR ($CDCl_3$);

| | |
|---|---|
| 0.92 (m, 6H, $CH_3$—C), | 1.04 (m, 3H, $CH_3$—C), |
| 1.24 (m, 28H, $CH_2$—C), | 2.38 (m, 1H, CH—C), |
| 3.55 (m, 2H, $CH_2$—O), | 4.22 (m, 1H, CH—O), |
| 5.20 (m, 1H, CH—O), | 6.04 (m, 1H, CH = C), |
| 6.78 (m, 1H, CH = C) | |

Preparation Example 2

In 50 ml of carbon tetrachloride was dissolved 3.8 g of 6-cetyloxy-2-isopropyl-2H-pyran-3(6H)-one with stirring. Into the solution was gradually introduced chlorine gas (0.71 g) at a temperature below 10°C and after the subsequent stirring for 30 minutes was added dropwise 0.8 g of pyridine maintaining the temperature below 15°C. Crystals were separated from the reaction mixture by suction filter. The filtrate was washed with 5% aqueous hydrochloric acid solution, saturated aqueous sodium hydrogencarbonate solution and saturated aqueous sodium chloride solution, and then dried over anhydrous magnesium sulfate. By the distillation of solvent was obtained 4.12 g of 6-cetyloxy-4-chloro-2-isopropyl-2H-pyran-3(6H)-one [compound 2] in a yellow viscous oily form.

IR (neat, $cm^{-1}$); 1690 (C = O), 1624 (C = C)
NMR ($CDCl_3$);

| | |
|---|---|
| 0.92 (m, 6H, $CH_3$—C), | 1.04 (m, 3H, $CH_3$—C), |
| 1.23 (m, 28H, $CH_2$—C), | 2.38 (m, 1H, CH—C), |
| 3.55 (m, 2H, $CH_2$—O), | 4.22 (m, 1H, CH—O), |
| 5.32 (m, 1H, CH—O), | 7.12 (m, 1H, CH = C) |

Preparation Example 3

In 50 ml of methanol was dissolved 3.8 g of 6-cetyloxy-2-isopropyl-2H-pyran-3(6H)-one with stirring. Into the solution were added 1.02 g of piperidine and 1.05 g of 37% formalin solution, and the mixture was stirred at 55 to 60°C for 7 hours. After the removal of methanol, the residue was separated by a silica gel column (developer: benzene/ethyl acetate = 5/1) to give 4.62 g of 6-cetyloxy-4-piperidinomethyl-2-isopropyl-2H-pyran-3(6H)-one [compound 3] in a yellowish brown viscous oily form.

IR (neat, $cm^{-1}$); 1698 (C = O), 1628 (C = C)

NMR ($CDCl_3$);

0.90 (m, 6H, $CH_3$—C), 0.98 (m, 3H, $CH_3$—C),
1.24 (m, 28H, $CH_2$—C), 1.46 (m, 6H, $CH_2$—C),
2.28 (m, 4H, $CH_2$—N), 2.38 (m, 1H, CH—C),
3.0 (m, 2H, $CH_2$—N), 3.52 (m, 2H, $CH_2$—C)
4.22 (m, 1H, CH—O), 5.20 (m, 1H, CH—O),
6.68 (m, 1H, CH = C)

In the following Table 1 were given characteristics of some of compounds of the invention which were prepared in a manner similar to Preparation Examples 1 to 3. In the Table, IR is shown by (neat, $cm^{-1}$) and NMR by ($CDCl_3$, δ).

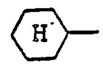

means a cyclohexyl group and Et means an ethyl group.

Table 1

| No. | Compound | Spectroscopic data |
|---|---|---|
| 4 | 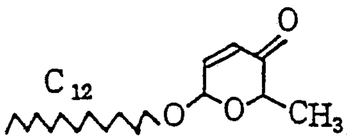 | IR ; 1698 (C=O), 1628 (C=C)<br>NMR ; 0.92 (m, 3H, $CH_3$-C),<br>1.22 (m, 3H, $CH_3$-C),<br>3.55 (m, 2H, $CH_2$-O),<br>4.22 (m, 1H, CH-O),<br>5.20 (m, 1H, CH-O),<br>6.04 (m, 1H, CH=C),<br>6.78 (m, 1H, CH=C) |
| 5 | 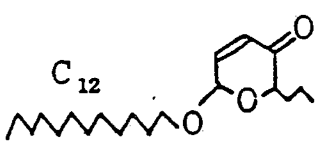 | IR ; 1698 (C=O), 1628 (C=C)<br>NMR ; 0.92 (m, 6H, $CH_3$-C),<br>1.24 (m, 24H, $CH_2$-C),<br>3.55 (m, 2H, $CH_2$-O),<br>4.22 (m, 1H, CH-O),<br>5.20 (m, 1H, CH-O),<br>6.04 (m, 1H, CH=C),<br>6.78 (m, 1H, CH=C) |

| | | |
|---|---|---|
| 6 | 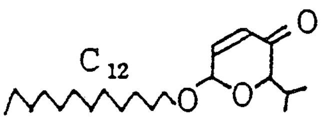 | IR ; 1698 (C=O), 1628 (C=C)<br>NMR ; 0.92 (m, 9H, $CH_3$-C),<br>1.24 (m, 20H, $CH_2$-C),<br>2.38 (m, 1H, CH-C),<br>3.55 (m, 2H, $CH_2$-O),<br>4.22 (m, 1H, CH-O),<br>5.20 (m, 1H, CH-O),<br>6.04 (m, 1H, CH=C),<br>6.78 (m, 1H, CH=C) |
| 7 | 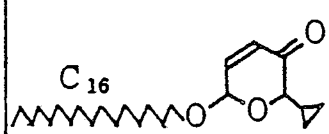 | IR ; 1698 (C=O), 1628 (C=C)<br>NMR ; 0.48 (m, 4H, $CH_2$-C),<br>0.90 (m, 3H, $CH_3$-C),<br>1.24 (m, 28H, $CH_2$-C),<br>1.68 (m, 1H, CH-C),<br>3.55 (m, 2H, $CH_2$-O),<br>4.22 (m, 1H, CH-O),<br>5.20 (m, 1H, CH-O),<br>6.04 (m, 1H, CH=C),<br>6.78 (m, 1H, CH=C) |

| | | |
|---|---|---|
| 8 | 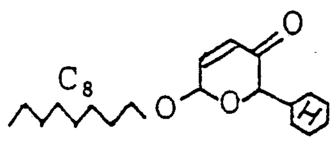 | IR ; 1698 (C=O), 1628 (C=C)<br>NMR ; 0.92 (m, 3H, $CH_3$-C),<br>0.7 - 1.9 (broad, 11H, (H)-),<br>1.24 (m, 12H, $CH_2$-C),<br>3.55 (m, 2H, $CH_2$-O),<br>4.22 (m, 1H, CH-O),<br>5.20 (m, 1H, CH-O),<br>6.04 (m, 1H, CH=C),<br>6.78 (m, 1H, CH=C) |
| 9 | 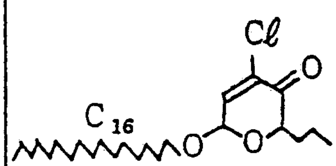 | IR ; 1690 (C=O), 1624 (C=C)<br>NMR ; 0.92 (m, 6H, $CH_3$-C),<br>1.24 (m, 32H, $CH_2$-C),<br>3.55 (m, 2H, $CH_2$-O),<br>4.22 (m, 1H, CH-O),<br>5.32 (m, 1H, CH-O),<br>7.12 (m, 1H, CH=C) |

| | | |
|---|---|---|
| 10 | 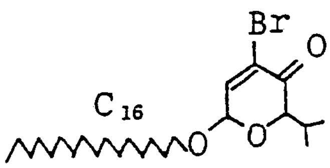 | IR ; 1690 (C=O), 1624<br>(C=C)<br>NMR ; 0.92 (m, 6H, $CH_3$-C),<br>1.04 (m, 3H, $CH_3$-C),<br>1.23 (m, 28H, $CH_2$-C),<br>2.38 (m, 1H, CH-C),<br>3.55 (m, 2H, $CH_2$-O),<br>4.22 (m, 1H, CH-O),<br>5.32 (m, 1H, CH-O),<br>7.12 (m, 1H, CH=C) |
| 11 | 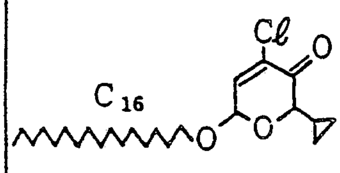 | IR ; 1690 (C=O), 1624<br>(C=C)<br>NMR ; 0.48 (m, 4H, $CH_2$-C),<br>0.90 (m, 3H, $CH_3$-C),<br>1.24 (m, 28H, $CH_2$-C),<br>1.68 (m, 1H, CH-C),<br>3.55 (m, 2H, $CH_2$-O),<br>4.22 (m, 1H, CH-O),<br>5.32 (m, 1H, CH-O),<br>7.12 (m, 1H, CH=C) |

| | | |
|---|---|---|
| 12 | 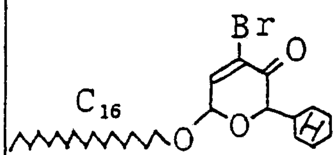 | IR ; 1690 (C=O), 1624 (C=C)<br>NMR ; 0.92 (m, 3H, $CH_3$-C),<br>0.7 - 1.9 (broad, 11H, 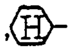-),<br>1.24 (m, 28H, $CH_2$-C),<br>3.55 (m, 2H, $CH_2$-O),<br>4.22 (m, 1H, CH-O),<br>5.32 (m, 1H, CH-O),<br>7.12 (m, 1H, CH=C) |
| 13 | 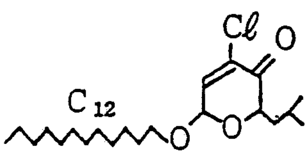 | IR ; 1690 (C=O), 1624 (C=C)<br>NMR ; 0.94 (m, 9H, $CH_3$-C),<br>1.28 (m, 22H, $CH_2$-C),<br>1.70 (m, 1H, CH-C),<br>3.55 (m, 2H, $CH_2$-O),<br>4.22 (m, 1H, CH-O),<br>5.32 (m, 1H, CH-O),<br>7.12 (m, 1H, CH=C) |

| | | |
|---|---|---|
| 14 | 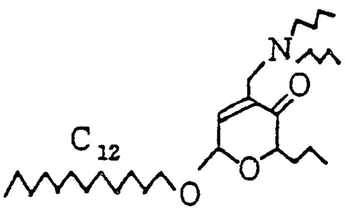 | IR ;1698(C=O),1628 (C=C)<br>NMR; 0.94(m,9H,$CH_3$-C),<br>1.26(m,32H,$CH_2$-C),<br>2.32(m,4H,$CH_2$-N),<br>3.02(m,2H,$CH_2$-N),<br>3.53(m,2H,$CH_2$-O),<br>4.22(m,1H,CH-O),<br>5.20(m,1H,CH-O),<br>6.68(m,1H,CH=C) |
| 15 | 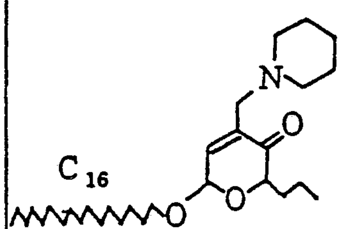 | IR ;1698(C=O),1628 (C=C)<br>NMR; 0.92(m,6H,$CH_3$-C),<br>1.24(m,32H,$CH_2$-C),<br>1.46(m,6H,$CH_2$-C),<br>2.28(m,4H,$CH_2$-N),<br>3.0 (m,2H,$CH_2$-N),<br>3.52(m,2H,$CH_2$-O),<br>4.22(m,1H,CH-O),<br>5.20(m,1H,CH-O),<br>6.68(m,1H,CH=C) |

| | | |
|---|---|---|
| 16 | 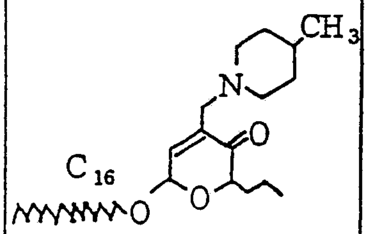 | IR ; 1698(C=O), 1628(C=C)<br>NMR ; 0.92 (m, 9H, $CH_3$-C),<br>1.24 (m, 32H, $CH_2$-C),<br>1.46 (m, 5H, $CH_2$-C),<br>2.30 (m, 4H, $CH_2$-N),<br>3.02 (m, 2H, $CH_2$-N),<br>3.52 (m, 2H, $CH_2$-O),<br>4.23 (m, 1H, CH-O),<br>5.20 (m, 1H, CH-O),<br>6.68 (m, 1H, CH=C) |
| 17 | 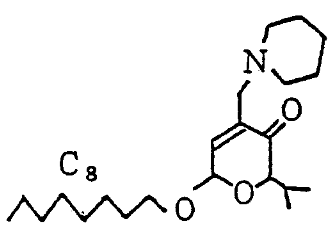 | IR ; 1698(C=O), 1628(C=C)<br>NMR ; 0.90 (m, 6H, $CH_3$-C),<br>0.98 (m, 3H, $CH_3$-C),<br>1.24 (m, 12H, $CH_2$-C),<br>1.46 (m, 6H, $CH_2$-C),<br>2.28 (m, 4H, $CH_2$-N),<br>2.38 (m, 1H, CH-C),<br>3.0 (m, 2H, $CH_2$-N),<br>3.52 (m, 2H, $CH_2$-O),<br>4.22 (m, 1H, CH-O),<br>5.20 (m, 1H, CH-O),<br>6.68 (m, 1H, CH=C) |

| | | |
|---|---|---|
| 18 | 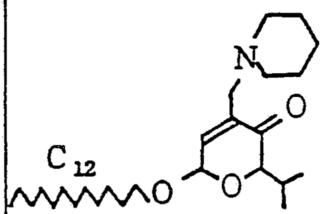 | IR ; 1698(C=O), 1628(C=C)<br>NMR ; 0.90 (m, 6H, $CH_3$-C),<br>0.98 (m, 3H, $CH_3$-C),<br>1.24 (m, 20H, $CH_2$-C),<br>1.46 (m, 6H, $CH_2$-C),<br>2.28 (m, 4H, $CH_2$-N),<br>2.38 (m, 1H, CH-C),<br>3.0 (m, 2H, $CH_2$-N),<br>3.52 (m, 2H, $CH_2$-O),<br>4.22 (m, 1H, CH-O),<br>5.20 (m, 1H, CH-O),<br>6.68 (m, 1H, CH=C) |
| 19 | 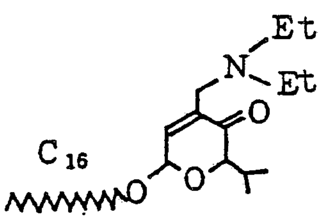 | IR ; 1698(C=O), 1628(C=C)<br>NMR ; 0.92 (m, 6H, $CH_3$-C),<br>0.98 (m, 3H, $CH_3$-C),<br>1.04 (m, 6H, $CH_3$-C),<br>1.24 (m, 28H, $CH_2$-C),<br>2.32 (m, 4H, $CH_2$-N),<br>2.38 (m, 1H, CH-C),<br>3.02 (m, 2H, $CH_2$-N),<br>3.52 (m, 2H, $CH_2$-O),<br>4.22 (m, 1H, CH-O),<br>5.20 (m, 1H, CH-O),<br>6.68 (m, 1H, CH=C) |

| | | |
|---|---|---|
| 20 | 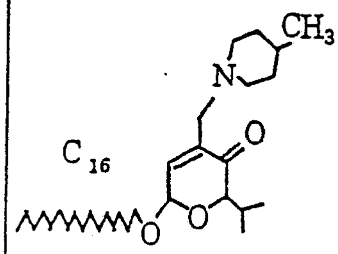 | IR ; 1698(C=O), 1628(C=C)<br>NMR ; 0.92 (m, 12H, $CH_3$-C),<br>1.24 (m, 28H, $CH_2$-C),<br>1.46 (m, 5H, $CH_2$-C),<br>2.28 (m, 4H, $CH_2$-N),<br>2.38 (m, 1H, CH-C),<br>3.02 (m, 2H, $CH_2$-N),<br>3.52 (m, 2H, $CH_2$-O),<br>4.23 (m, 1H, CH-O),<br>5.20 (m, 1H, CH-O),<br>6.68 (m, 1H, CH=C) |
| 21 | 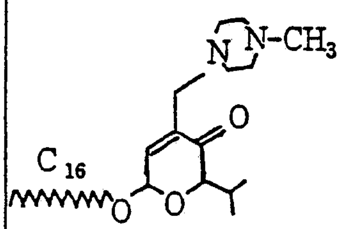 | IR ; 1698(C=O), 1628(C=C)<br>NMR ; 0.92 (m, 9H, $CH_3$-C),<br>1.24 (m, 28H, $CH_2$-C),<br>2.22 (s, 3H, $CH_3$-N),<br>2.28 (m, 4H, $CH_2$-N),<br>2.30 (m, 4H, $CH_2$-N),<br>2.38 (m, 1H, CH-C),<br>3.02 (m, 2H, $CH_2$-N),<br>3.52 (m, 2H, $CH_2$-O),<br>4.23 (m, 1H, CH-O),<br>5.20 (m, 1H, CH-O),<br>6.68 (m, 1H, CH=C) |

| | | |
|---|---|---|
| 22 | 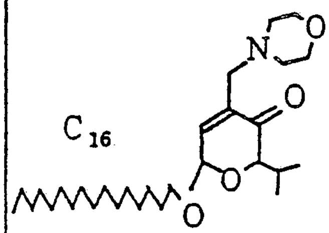 | IR ; 1698(C=O), 1628(C=C)<br>NMR ; 0.90 (m, 6H, $CH_3$-C),<br>0.98 (m, 3H, $CH_3$-C),<br>1.24 (m, 28H, $CH_2$-C),<br>2.38 (m, 1H, CH-C),<br>2.42 (m, 4H, $CH_2$-N),<br>3.08 (m, 2H, $CH_2$-N),<br>3.52 (m, 2H, $CH_2$-O),<br>3.65 (m, 4H, $CH_2$-O),<br>4.22 (m, 1H, CH-O),<br>5.20 (m, 1H, CH-O),<br>6.68 (m, 1H, CH=C) |
| 23 | 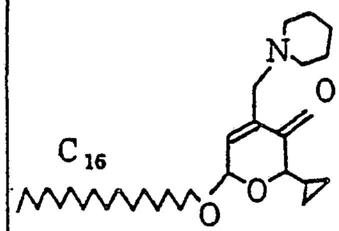 | IR ; 1698(C=O), 1628(C=C)<br>NMR ; 0.48 (m, 4H, $CH_2$-C),<br>0.90 (m, 3H, $CH_3$-C),<br>1.24 (m, 28H, $CH_2$-C),<br>1.46 (m, 6H, $CH_2$-C),<br>1.68 (m, 1H, CH-C),<br>2.28 (m, 4H, $CH_2$-N),<br>3.0 (m, 2H, $CH_2$-N),<br>3.52 (m, 2H, $CH_2$-O),<br>4.22 (m, 1H, CH-O),<br>5.20 (m, 1H, CH-O),<br>6.68 (m, 1H, CH=C) |

| | | |
|---|---|---|
| 24 | 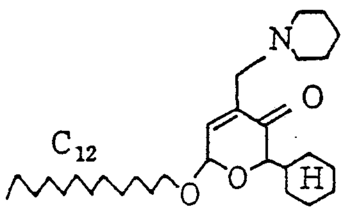 | IR ; 1698(C=O), 1628(C=C)<br>NMR ; 0.90 (m, 3H, $CH_3$-C),<br>0.7 - 1.9 (broad, 11H, 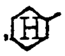),<br>1.24 (m, 20H, $CH_2$-C),<br>1.46 (m, 6H, $CH_2$-C),<br>2.28 (m, 4H, $CH_2$-N),<br>3.0 (m, 2H, $CH_2$-N),<br>3.52 (m, 2H, $CH_2$-O),<br>4.22 (m, 1H, CH-O),<br>5.20 (m, 1H, CH-O),<br>6.68 (m, 1H, CH=C) |
| 25 | 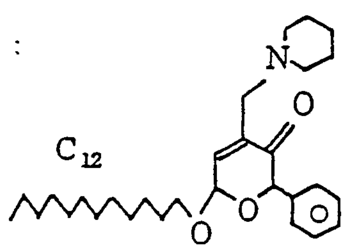 | IR ; 1698(C=O), 1628(C=C)<br>NMR ; 0.90 (m, 3H, $CH_3$-C),<br>1.24 (m, 20H, $CH_2$-C),<br>1.46 (m, 6H, $CH_2$-C),<br>2.28 (m, 4H, $CH_2$-N),<br>3.0 (m, 2H, $CH_2$-N),<br>3.52 (m, 2H, $CH_2$-O),<br>4.22 (m, 1H, CH-O),<br>5.20 (m, 1H, CH-O),<br>6.68 (m, 1H, CH=C),<br>7.36 (s, 5H, 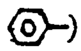) |

Test Example 1

Twenty female adults of Tetranychus urticae were incubated on kidney beans (2-week-old seedlings) in each of two pots. A solution having a specified concentration was prepared by diluting 50% emulsion containing one of the present compounds. The solution was applied to the kidney bean seedlings to sufficiently wet the leaf surfaces. The number of the survivals was counted in 3 days. The mortality is shown in Table 2. The same procedure as above was repeated with use of the other compounds. For comparison, the results with a control drug and with no drug are also shown in the Table. As control drug is used Kelthane® (trade name, a product of Röhm & Haas Co., Ltd.).

Table 2

| Compound | Concentration of effective compound | |
|---|---|---|
| | 300 ppm | 150 ppm |
| 1 | 100% | 100% |
| 2 | 100% | 97% |
| 3 | 83% | 75% |
| 7 | 100% | 100% |
| 18 | 100% | 90% |
| Control | 100% | 75% |
| No drug | 0% | 0% |

Test Example 2

Female adults of Panonychus citri were incubated on leaves of bitter oranges for 2 days and allowed to lay eggs. The eggs as deposited on the leaves (50 leaves) were immersed for 10 seconds in a solution having a specified concentration and prepared by diluting 50% emulsion containing one of the present compounds and dried in air.

Thereafter, the eggs and leaves were placed in a chamber maintained at 27°C without allowing them to dry. The number of hatched eggs was counted in 7 days to calculate the ratio of killed eggs. The same procedure as above was repeated with use of the other compounds. Table 3 shows the results with a control drug (Zardex®, trade name, a product of Zoecon Co., Ltd.) and with no drug.

Table 3

| Compound | Concentration of effective compound | |
|---|---|---|
| | 200 ppm | 100 ppm |
| 1 | 100% | 100% |
| 3 | 100% | 90% |
| 15 | 97% | 95% |
| 18 | 100% | 97% |
| 23 | 90% | 85% |
| Control | 87% | 79% |
| No drug | 0% | 0% |

**Claims**

1. A pyran derivative represented by a general formula

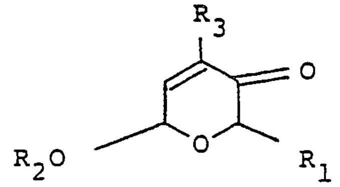

(I)

wherein $R_1$ is hydrogen, lower alkyl of 1 to 6 carbon atoms, cycloalkyl of 3 to 7 carbon atoms or phenyl, $R_2$ is

alkyl of 8 to 20 carbon atoms, $R_3$ is hydrogen, halogen,

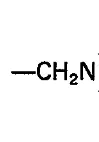 or $-CH_2\widehat{NA}$,

$R_4$ and $R_5$ being each lower alkyl of 1 to 6 carbon atoms or cycloalkyl of 3 to 7 carbon atoms, A being $-CH_2-$, an oxygen or nitrogen atom,

$\widehat{NA}$

forms a five or six-membered ring (the ring may have no substituent or have at least one substituent selected from lower alkyl of 1 to 6 carbon atoms, lower alkoxy of 1 to 6 carbon atoms and halogen), and an acid salt thereof.

2. A pyran derivative and an acid salt thereof as defined in claim 1, wherein $R_3$ is

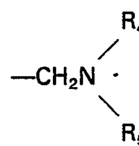 ,

$R_4$ and $R_5$ being each lower alkyl of 1 to 6 carbon atoms.

3. A pyran derivative and an acid salt thereof as defined in claim 1, wherein $R_3$ is

$-CH_2\widehat{NB}$,

B being $-CH_2-$ or an oxygen atom.

4. A process for preparing a pyran derivative according to claim 1, which comprises reacting a pyran derivative of the general formula

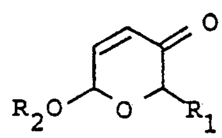

wherein $R_1$ and $R_2$ are as defined above, with formalin and an amine selected from

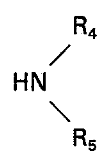

and $H\widehat{NA}$, wherein $R_4$, $R_5$ and A are as defined above, in a lower alcohol.

5. A miticide which comprises a pyran derivative according to claim 1 or an acid salt thereof.

**Patentansprüche**

1. Pyranderivat der allgemeinen Formel

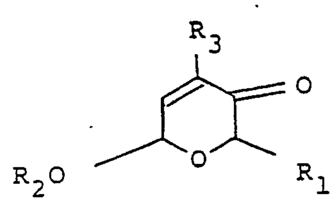

(I)

worin $R_1$ Wasserstoff, Niederalkyl mit 1 bis 6 Kohlenstoffatomen, Cycloalkyl mit 3 bis 7 Kohlenstoffatomen

oder Phenyl ist, $R_2$ Alkyl mit 8 bis 20 Kohlenstoffatomen ist, $R_3$ Wasserstoff, Halogen,

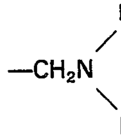 oder 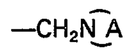

ist, wobei $R_4$ und $R_5$ jeweils Niederalkyl mit 1 bis 6 Kohlenstoffatomen oder Cycloalkyl mit 3 bis 7 Kohlenstoffatomen sind, A $-CH_2-$, ein Sauerstoff- oder Stickstoffatom ist,

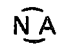

einen fünf- oder sechsgliedrigen Ring bildet (der Ring kann keinen Substituenten oder mindestens einen Substituenten, ausgewählt unter Niederalkyl mit 1 bis 6 Kohlenstoffatomen, Niederalkoxy mit 1 bis 6 Kohlenstoffatomen und Halogen, aufweisen) und dessen Säuresalze.

2. Pyranderivat und dessen Säuresalze nach Anspruch 1, worin $R_3$

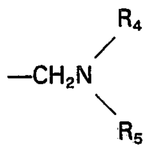

ist, wobei $R_4$ und $R_5$ jeweils Niederalkyl mit 1 bis 6 Kohlenstoffatomen sind.

3. Pyranderivat und dessen Säuresalze nach Anspruch 1, worin $R_3$

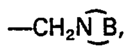,

ist, wobei B $-CH_2-$ oder ein Sauerstoffatom ist.

4. Verfahren zur Herstellung eines Pyranderivats nach Anspruch 1, welches umfaßt die Umsetzung eines Pyranderivats der allgemeinen Formel

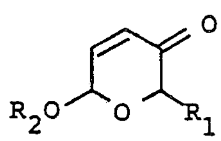

worin $R_1$ und $R_2$ wie oben definiert sind, mit Formalin und einem Amin, ausgewählt unter

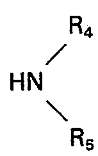 und 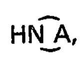,

worin $R_4$, $R_5$ und A wie oben definiert sind, in einem niederen Alkohol.

5. Miticid, welches ein Pyranderivat nach Anspruch 1 oder ein Säuresalz davon umfaßt.

**Revendications**

1. Les dérivés du pyranne de formule générale ci-dessous:

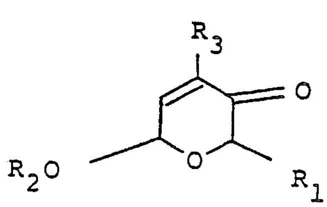

(I)

(dans laquelle $R_1$ représente l'hydrogène, un alkyle en $C_1$ à $C_6$, un cycloalkyle en $C_3$ à $C_7$ ou un phényle, $R_2$ un alkyle en $C_8$ à $C_{10}$ et $R_3$ l'hydrogène, un halogène ou un groupe

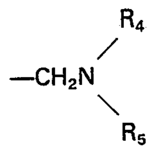 ou 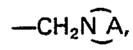

$R_4$ et $R_5$ étant chacun un alkyle en $C_1$—$C_6$ ou un cycloalkyle en $C_3$ à $C_7$, A désignant le groupe —$CH_2$— ou un atome d'oxygène ou d'azote et

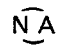

formant un cycle pentagonal ou hexagonal sans substituants ou avec un ou plusieurs substituants choisis parmi des alkyles et des alcoxy en $C_1$ à $C_6$ et les halogènes), ainsi que des sels d'acides de ces composés.

2. Un dérivé du pyranne ou un sel d'acide de celui-ci, tel que défini à la revendication 1, dans lequel $R_3$ est un groupe

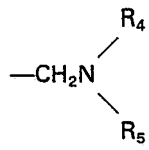 ,

$R_4$ et $R_5$ étant chacun un alkyle en $C_1$—$C_6$.

3. Un dérivé du pyranne ou un sel d'acide de celui-ci, tel que défini à la revendication 1, dans lequel $R_3$ est un groupe

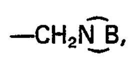

B étant un groupe —$CH_2$— ou un atome d'oxygène.

4. Un procédé de préparation des dérivés du pyranne selon la revendication 1, procédé selon lequel on fait réagir un dérivé de pyranne de formule générale:

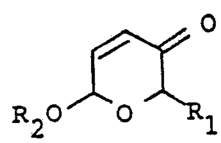

avec du formaldéhyde et une amine

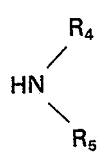 ou 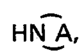

$R_4$, $R_5$ et A ayant les significations données, dans un alcool inférieur.

5. Un produit miticide comprenant comme matière active un dérivé du pyranne selon la revendication 1 ou un sel d'acide de ce dérivé.